# EUROPEAN PATENT APPLICATION

(11) **EP 4 821 912 A1**
(43) Date of publication of application: **14.10.2026**
(21) Application number: 25305526.3
(22) Date of filing: 11.04.2025
(51) Int. Cl.: C07C 213/06, C07C 219/08

(54) **PROCESS FOR A CONTINUOUS SYNTHESIS OF AMINO ALKYL (METH)ACRYLATES**

(71) Applicant: SNF Group, 42160 Andrézieux-Bouthéon (FR)
(72) Inventor: KIEFFER, Johann, 42160 ANDREZIEUX-BOUTHEON (FR); LEGRAS, Benoît, 42160 ANDREZIEUX-BOUTHEON (FR); HOVASSE, Nicolas, 42160 ANDREZIEUX-BOUTHEON (FR); FAVERO, Cédrick, 42160 ANDREZIEUX-BOUTHEON (FR)
(74) Representative: Ipsilon

(57) **Abstract**

The present invention relates to a new amino alkyl (meth)acrylate continuous synthesis process.

## Description

### TECHNICAL FIELD OF THE INVENTION

The present invention relates to a new amino alkyl (meth)acrylates continuous synthesis process.

### TECHNICAL BACKGROUND

Amino alkyl (meth)acrylates, such as 2-dimethylaminoethyl (meth)acrylate and its quaternized derivatives, are well-known monomers used in the preparation of polymers used in many industries, such as water treatment, papermaking, home and personal care, or oil & gas industry such as enhanced oil recovery, hydraulic fracturing, water shut-off and others.

These monomers are obtained by transesterification of alkyl(meth)acrylate with an alkanolamine. Catalysts for this transesterification reaction include organometallic compounds. Choosing the catalyst depends on various criteria, for instance the nature of the amino alkyl (meth)acrylate or of the alkanolamine, or the nature of the synthesis process. The catalyst allows displacing the equilibrium to produce more amino alkyl (meth)acrylate and reduces the formation of impurities.

When the transesterification reaction ends, the catalyst can be recycled in a new synthesis process. During these recycling cycles, the catalyst tends to be deactivated leading to the formation of more impurities. These impurities, called heavies, are Michael adducts of alcohols and/or amine, such as methanol or dimethylaminoethanol which react on the double bond of the methyl(meth)acrylate, dimethylaminoethyl(meth)acrylate, or (meth)acrylic acid. The formation of these impurities reduces the yield of the transesterification reaction, increases the wastes generated and decreases the quality of the amino alkyl (meth)acrylate produced, which impacts the performances of polymers prepared from such amino alkyl (meth)acrylate.

Many attempts have been made to reduce the formation of heavies. WO 2019/196048 suggests replacing part of the original catalyst with a fresh one in the successive synthesis batch, leading to a better lifetime of the recycled catalyst and better yield and purity of the amino alkyl (meth)acrylate produced. However, this solution requires an increase in catalyst consumption.

U S 4,059,617 describes the synthesis of dimethylaminoethyl methacrylate in batch process wherein a mixture of dimethylaminoethanol and methylmethacrylate is continuously added as the aminoalcohol reactant is consumed. As a result, the yield is improved and less heavies are formed.

Alternative solutions are disclosed in WO 2013/045786 and WO 2016/124837. In these documents heavies undergo thermal cracking in order to reverse their formation. However, such a process requires a lot of energy.

The Applicant has discovered that it is possible to reduce the formation of heavies and thus improve the conversion rate of raw materials to amino alkyl (meth)acrylate during a continuous synthesis process. Accordingly, the invention reduces the wastes generated by separating the continuous synthesis in a minimum of two reactive devices, wherein the alkanolamine is added to the two distillation devices, in a specific ratio. Since less heavies are obtained, the recuperation of the amino alkyl (meth)acrylate by distillation requests less time, which leads to a reduction of the overall amount of energy required for the amino alkyl (meth)acrylates recuperation. As a result, the invention reduces the overall emission of greenhouse gases, such as CO₂, associated to amino alkyl (meth)acrylates synthesis process.

The aim of the invention is to reduce the heavies' formation compared to the amino alkyl (meth)acrylate produced. This is in line with the second principle of green chemistry - atom economy. The process according to the invention makes it possible to reduce the E Factor, which is the measure of the total quantity of waste produced by a reaction in relation to the quantity of final product.

The present invention is advantageously carried out using materials of biological origin (such as biomass), or recycled materials. The synthesis of the compounds used in the invention are advantageously a biological synthesis, for example, by enzymatic catalysis or extracted from renewable raw material. The energy used to carry out the process according to the invention is advantageously derived from a heat pump or of renewable origin, for example wind power, photovoltaics, or, in particular for mobile installations, from fuel cell or lithium battery type.

### SUMMARY OF THE INVENTION

The present invention relates to a new amino alkyl (meth)acrylates continuous synthesis process.

More specifically, the continuous synthesis process of amino alkyl (meth)acrylate AAE2 comprises a continuous transesterification reaction from at least one alkanolamine AKN1 and at least one alkyl(meth)acrylate ester AAE1 in at least one solvent in presence of at least one catalyst C1;
the reaction is carried out in at least two distillation devices put in series.

### DESCRIPTION OF THE INVENTION

By "polymer" we designate a homopolymer or a copolymer. A homopolymer is a polymer composed of a single identical repeating unit, while a copolymer is composed of two or more different repeating units.

As used herein, the expression "A and/or B" means "A, or B, or A and B".

The invention also includes all possible combinations of the various embodiments disclosed, whether they are preferred or exemplary embodiments when they are not mutually exclusive. Furthermore, where ranges of values are indicated, the end-values are part of these ranges. The disclosure also includes all combinations between the end-values of these ranges. For example, ranges "1-20, preferably 5-15", imply disclosure of the ranges "1-5", "1-15", "5-20" and "15-20".

All the particular and/or preferred embodiments described in the invention are combinable, provided they are not incompatible.

### Continuous transesterification reaction

The continuous synthesis process of amino alkyl (meth)acrylate AAE2 comprises a continuous transesterification reaction from at least one alkanolamine AKN1 and at least one alkyl(meth)acrylate ester AAE1 in at least one solvent S1 in presence of at least one catalyst C1, the reaction is carried out in at least two distillation devices put in series (DD1 and DD2) and a continuous distillate is removed from the at least two distillation devices.

More specifically, the invention relates to a process for the continuous synthesis of an amino alkyl (meth)acrylate AAE2 wherein the process comprises continuous transesterification reactions carried out in a first distillation device (DD1) and in a second distillation device (DD2), wherein the first and the second distillation devices DD1 and DD2 are arranged in series,
wherein the process comprises the following steps:
1/ adding to the first and the second distillation devices DD1 and DD2, a mixture M1 comprising an alkyl(meth)acrylate ester AAE1, a solvent S1, an alkanolamine AKN1 and a catalyst C1;
2/ transesterification reactions, in the first and second distillation devices DD1 and DD2, of the alkyl(meth)acrylate ester AAE1 with the alkanolamine AKN1 while continuously distilling an azeotropic mixture of solvent S1 and an alkyl alcohol formed during the transesterification reactions,
   wherein the transesterification reactions afford an amino alkyl(meth)acrylate AAE2 and are carried out at a temperature of between 40 and 120°C; and,
   wherein the first distillation device DD1 is continuously fed with a mixture M2 comprising the alkyl(meth)acrylate ester AAE1, the solvent S1, the alkanolamine AKN1 and the catalyst C1; and,
   wherein the second distillation device DD2 is continuously fed with the alkanolamine AKN1; and,
   wherein the weight ratio between the alkanolamine AKN1 fed to the first distillation device DD1 and the alkanolamine AKN1 fed to the second distillation device DD2 is comprised between 0.4:1 and 6:1, based on the total amount of alkanolamine AKN1 fed in the transesterification reactions; and,
   wherein during the transesterification reaction, an overflow of the first distillation device DD1 fills the second distillation device DD2.

The alkanolamine(s) AKN1, the alkyl(meth)acrylate(s) ester AAE1, the solvent(s) S1 and the catalyst(s) C1 are added in the distillation device DD1 and then an overflow of the first distillation device DD1 fills the second distillation device DD2 and the overflow of distillation device DD2 is recovered in a recuperation device RD.

The distillation devices can be any distillation device known from the skilled man in the art and able to evaporate the azeotrope formed between the solvent and the alkyl alcohol resulting from the transesterification of the alkyl (meth)acrylate ester AAE1 with the alkanolamine AKN1. For example, the process can be carried out in various types of equipment, including, but not limited to, multiple distillation columns with integrated reactors, which may function as reboilers. These reactors can be mechanically stirred or non-mechanically stirred. In cases where mechanical stirring is not employed, examples of suitable reactors include tubular micro-reactors equipped with mixing elements, or microplate reactors. Other configurations may include distillation columns with stacked reactors, reactive distillation columns with membrane separation, or reactive distillation columns with integrated heat exchangers. Preferably, the equipment is a distillation column equipped with stirred reactors.

The distillation devices DD1 and DD2 can be the same equipment type, or they can be different. Preferably, they are the same.

The transesterification reaction in distillation devices DD1 is carried out at a temperature of between 40 and 120°C, preferably between 70 and 100°C.

The transesterification reaction in distillation devices DD2 is carried out at a temperature comprised between 40 and 120°C, preferably between 70 and 100°C.

The transesterification reaction in distillation devices DD1 lasts advantageously between 30 minutes and 720 minutes, preferably between 240 minutes and 500 minutes.

The transesterification reaction in distillation devices DD2 lasts advantageously between 30 minutes and 720 minutes, preferably between 240 minutes and 500 minutes.

The transesterification reaction in distillation devices DD1 can be carried out at atmospheric pressure, at a pressure below or above the atmospheric pressure. Preferably, the distillation is carried out at above the atmospheric pressure.

In a particular embodiment, the catalyst C1 is an enzymatic catalyst. In this case, the transesterification reaction in the distillation devices DD1 is preferably carried out at a pressure below atmospheric pressure.

The transesterification reaction in distillation devices DD1 is preferably carried out at a pressure of between 1 mbar and 2 bar relative (1 mbar = 100 Pa), preferably between 50 mbar and 1.8 bar relative, more preferably between 100 mbar relative and 1.5, even more preferably less than 200 mbar and 1.2 bar relative. Relative pressure corresponds to pressure relative to atmospheric pressure (1 bar).

The transesterification reaction in distillation devices DD2 can be carried out at atmospheric pressure, or at a pressure below the atmospheric pressure. Preferably, the distillation is carried out at below the atmospheric pressure.

In a particular embodiment, the catalyst C1 is an enzymatic catalyst, in this case the transesterification reaction in the distillation devices DD2 is carried out at pressure below the atmospheric pressure. The transesterification reaction in distillation devices DD2 is generally carried out at a pressure of between 1 mbar and 2 bar relative (1 mbar = 100 Pa), preferably between 50 mbar and 1.8 bar relative, more preferably between 100 mbar relative and 1.5, even more preferably less than 200 mbar and 1.2 bar relative. Relative pressure corresponds to pressure relative to atmospheric pressure (1 bar).

By distilling the azeotrope formed between the solvent and the alkyl alcohol resulting from the transesterification of the alkyl (meth)acrylate ester AAE1 with the alkanolamine AKN1, the thermodynamic equilibrium is shifted toward the formation of the amino alkyl (meth)acrylates.

The alkyl (meth)acrylate ester AAE1 may be linear, cyclic (substituted or not) or branched. Preferably, the alkyl (meth)acrylate ester AAE1 is linear and the alkyl chain comprises advantageously from 1 to 10 carbons, preferably between 1 and 8, more preferably between 1 and 4. Even more preferably, the alkyl group is methyl or ethyl. Even more preferentially, the alkyl (meth)acrylate AAE1 is methyl(meth)acrylate.

The alkanolamine AKN1 corresponds to a chemical compound that contains both hydroxyl (-OH) and amine functional groups on a hydrocarbonated backbone. The amine functional group may be a primary (-NH₂), secondary (-NHR¹), or tertiary (-NR¹R²) amine. Preferably the amine functional group is a tertiary amine (-NR¹R²). The hydrocarbonated backbone is a linear or branched hydrocarbon chain, which preferably comprises between 1 and 10 carbon atoms.

The alkyl radicals of the tertiary amine -R¹ and -R² are preferably hydrocarbon chains comprising each, independently from each other, between 1 and 8 carbon atoms, preferably between 1 and 4 carbon atoms. More preferably, -R¹ and -R² have each 1 carbon atom (-CH₃ group).

The quantity of catalyst C1 is advantageously comprised between 0.1 and 150 mmol, per mol of alkanolamine AKN1, based on the total amount of alkanolamine AKN1 used in the continuous synthesis process (total amount in all the distillation devices, which is preferably the amount added in DD1 + amount added in DD2 when the process only involves the first and the second distillation devices DD1 and DD2), preferably between 1 and 30 mmol, more preferably between 1.1 and 25 mmol.

The catalyst C1 is preferably an organometallic compound. For example, the organometallic can be a compound of tin, titanium, zirconium or zinc. Catalysts include (but are not limited to) titanium tetra isopropoxyde (TTIP), dibutyltin oxide (DBTO) and zirconium tetraacetylacetonate. Preferably, the catalyst C1 is a derivate of tin, more preferably a dialkyltin oxide. Even more preferably, the catalyst C1 is dibutyltin oxide (DBTO).

In a preferred embodiment, the catalyst C1 is an enzyme or a mixture of enzymes able to catalyze a transesterification reaction, for example, a lipase. Preferably, the lipase is selected from the group consisting of: Lipozyme^{®} TL IM, Lipozyme^{®} CALB L, Lipozyme^{®}TL 100 L, NovoCor^{®} AD L, Novozym^{®} 435, Lipozyme^{®} RM, Novozym^{®} 51032, Paltase^{®} 20000 L, and Resinase^{®} HT.

In a preferred embodiment, the catalyst C1 is added in the form of a suspension in solvent S1 in which 70% to 95% by weight of the catalyst C1 is solubilized, and 5% to 30% by weight of catalyst C1 is in the form of particles suspended in the suspension S1, based on the total amount of catalyst C1 in solvent S1. Preferably, more than 80% and no more than 95%, more preferably more than 85% and no more than 95% by weight of catalyst C1 is solubilized in solvent S1.

The molar ratio between the alkyl (meth)acrylate ester AAE1 and the total amount of alkanolamine AKN1 used in the continuous synthesis process (total amount in all the distillation devices, which is preferably the amount added in DD1 + amount added in DD2 when the process only involves the first and the second distillation devices DD1 and DD2) is advantageously comprised between 1:5 and 5:1, preferably between 1:2 and 2:1.

The solvent S1 used in the process of the invention is advantageously an inert solvent, i.e. a solvent that does not react with the alkanolamine AKN1 and the alkyl (meth)acrylate ester AAE1. Solvent S1 is advantageously selected from the group consisting of C3-C16 linear hydrocarbons, C3-C16 branched hydrocarbons, C5-C16 cyclic hydrocarbons, and mixtures thereof. Solvent S1 can be saturated or unsaturated. It preferably consists of hydrogen atoms and carbon atoms. Preferably, the solvent is selected from n-hexane, the isomers of n-hexane, and mixtures thereof.

The ratio in weight between the solvent S1 and the total amount of alkanolamine AKN1, alkyl(meth)acrylate ester AAE1 and catalyst C1 used in the continuous synthesis process is advantageously comprised between 0.0001:1 and 0.3:1 (S1/(AKN1+AAE1+C1)), preferably between 0.001:1 and 0.2:1; more preferably between 0.01:1 and 0.1: 1

Between 25 and 90 by weight % of the at least alkanolamine AKN1, based on the total amount of alkanolamine AKN1 used in the continuous transesterification reaction, is preferably fed to the first distillation device DD1, and between 10 and 75% by weight of the at least alkanolamine AKN1, based on the total amount of alkanolamine AKN1 used in the continuous transesterification reaction, is preferably fed to the second distillation device DD2. In this case, the process preferably involves only two distillation devices, DD1 and DD2.

Between 25 and 90 by weight % of the at least alkanolamine AKN1, based on the total amount of alkanolamine AKN1 used in the continuous transesterification reaction, is preferably fed to the first distillation device DD1, preferably between 50 and 75 by weight %. In this case, the process preferably involves only two distillation devices, DD1 and DD2.

Between 10 and 75 by weight % of the at least alkanolamine AKN1, based on the total amount of alkanolamine AKN1 used in the continuous transesterification reaction, is preferably fed to the second distillation device DD2, preferably between 25 and 50 by weight %. In this case, the process preferably involves only two distillation devices, DD1 and DD2.

The ratio between the alkanolamine AKN1 added to the first distillation device DD1 and the alkanolamine AKN1 added to the second distillation device DD2 only impacts the kinetics of the continuous synthesis process of amino alkyl (meth)acrylates.

The overflow of distillation device DD2 going in the recuperation device RD is preferably distilled (D3).

The distillation D3 can be carried out in batch, semi-continuously or continuously.

The distillation D3 can be carried out in any distillation device known by the skilled man in the art and able to evaporate the amino alkyl(meth)acrylate ester resulting from the transesterification reaction in distillation devices DD2. For example, the process can be carried out in any one of various types of equipment, including, but not limited to, multiple distillation columns with integrated reactors, which may function as reboilers. These reactors can be mechanically stirred or non-mechanically stirred. In the absence of mechanical stirring, examples of suitable reactors include tubular micro-reactors equipped with mixing elements, or microplate reactors. Other configurations may include distillation columns with stacked reactors, reactive distillation columns with membrane separation, or reactive distillation columns with integrated heat exchangers. Preferably, the equipment is a distillation column equipped with stirred reactors.

The distillation D3 can be carried out in one or more distillation devices.

The distillation D3 can be carried out at atmospheric pressure, or at a pressure below the atmospheric pressure. Preferably, the distillation is carried out at below the atmospheric pressure.

When the distillation D3 is carried out at a pressure below the atmospheric pressure, the pressure is advantageously between 1 and less than 1000 mbar relative (1 mbar = 100 Pa). The pressure is preferably less than 900 mbar relative, more preferably less than 800 mbar relative, more preferably less than 700 mbar relative, more preferably less than 600 mbar relative, more preferably less than 500 mbar relative, more preferably less than 400 mbar relative, more preferably less than 300 mbar relative, more preferably less than 200 mbar relative, more preferably less than 100 mbar relative and even more preferably less than 50 mbar relative, and advantageously more than 1 mbar relative. Relative pressure corresponds to pressure relative to atmospheric pressure (1 bar). The distillation D3 lasts advantageously between 1 minutes and 600 minutes, preferably between 60 minutes and 360 minutes
To accelerate the distillation D3, the overflow of distillation device DD2 in the recuperation device RD can be heated, advantageously between 60 and 120 °C, preferably between 80 and 100°C.

The weight ratio between the alkanolamine AKN1 fed to the first distillation device DD1 and the alkanolamine AKN1 fed to the second distillation device DD2 is comprised between 0.4:1 and 6:1, based on the total amount of alkanolamine AKN1 fed in the transesterification reactions, preferably between 0.7:1 and 5:1 and more preferably between 1:1 and 4:1.

The process can be carried out in a main device comprising the first distillation device (DD1), the second distillation device (DD2) and n additional distillation devices (DDn+2) arranged in series, the last distillation device being connected to the recuperation (recovery) device RD, wherein n is an integer from 0 to 8, for instance from 1 to 8. The main device can therefore comprise n distillation devices DD arranged in series: DD1-DD2-n(DD).

When n is 1, the third distillation device is connected to the second distillation device: DD1-DD2-DD3. When n is 2, the third distillation device is connected to the second distillation device, wherein the fourth distillation device is connected to the third device: DD1-DD2-DD3-DD4. The transesterification reaction is carried out in all n+2 distillation devices. Each n additional distillation device (DDn+2) is fed with the overflow of the distillation device (DDn+1) while the alkanolamine AKN1 is distributed in all the distillation device calculated as: $% of alkanolamine in distillation device ′ m ′ = \frac{{exp}^{\left(∝∗\left(total number of distillation devices-m\right)\right.}}{{\sum }_{1}^{m} {exp}^{\left(∝∗\left(total number of distillation devices-m\right)\right.}}$ wherein m is an integer from 1 to 10.

The value of α is between 0 and 3, preferably between 0.2 and 2.5, and more preferably between 0.4 and 2.

The parameter α represents the distribution gradient of AKN1 between the distillation devices and is linked to the reaction kinetics and the residence time within these reactors.

The value of α increases as the residence times in the distillation devices become longer, indicating a higher distribution gradient.

The parameter α is determined based on the reaction kinetics and the residence time within the distillation or reaction devices. It may be obtained by empirical measurement or by process modelling or adjusted empirically in order to optimize the distribution of AKN1 and improve process performance. Higher residence times or faster kinetics typically lead to higher α values, resulting in a steeper distribution gradient.

In a particular embodiment, the invention can comprise p maturation devices MD after DD2 or after DDn+2, p is an integer from 1 to 8.

The maturation device preferably corresponds to the distillation devices previously described in which no alkanolamine AKN1 is continuously added.

When additional distillation device(s) are used, the conditions applied to DD1 and DD2 can also be applied, independently, to distillation devices DDn+2.

The skilled man in the art is able to determine the α value with simple routine tests.

### Optional step(s)

The continuous synthesis process according to the invention can comprise additional optional step(s). For example, after its recuperation, the amino alkyl (meth)acrylate AAE2 can be purified, for example by distillation.

When using an organotin catalyst, the latter can be recycled as described in patent application WO2019196048, or it can be recycled as described in patent application CN117000313.

In a preferred embodiment, at least one of the alkyl (meth)acrylate ester AAE1, the alkanolamine AKN1 and the solvent, of the continuous synthesis process according to the invention, originates from a feedstock comprising at least partially renewable and non-fossil origin.

In the context of the invention, the terms "of renewable and non-fossil origin" designate the origin of a chemical compound issuing from biomass or synthetic gas (syngas), namely, one that is the result of one or more chemical transformations performed on one or more raw materials of natural, and non-fossil, origin. The terms "bio-sourced" or "bio-resourced" can also be used to characterize the renewable and non-fossil origin of a chemical compound. The renewable and non-fossil origin of a compound includes renewable and non-fossil raw materials coming from the circular economy, and which have been previously recycled, one or more times, during a recycling process of material coming from biomass, such as, for example, material coming from polymer depolymerization or from the transformation of pyrolysis oil.

According to the invention, "at least partially of renewable and non-fossil origin" means a content of bio-sourced carbon comprised between 5% by weight and 100% by weight based on to the total carbon weight of said compound, preferably 30% to 100%, more preferably 50% to 100%, even more preferably 70% to 100%, more preferably 90% to 100%, and even more preferably, 100% bio-sourced carbon.

In the context of the invention, the standard ASTM D6866-21, method B, is used to characterize the bio-sourced nature of a chemical compound, and to determine the bio-sourced content of said compound. The value is expressed in percent by weight of bio-sourced carbon based on to the total weight of carbon in said compound.

The ASTM D6866-21 standard is a test method that teaches how to experimentally measure the bio-sourced carbon content of solids, liquids and gaseous samples by radiocarbon analysis.

In a particularly preferred embodiment, the alkyl (meth)acrylate ester AAE1, the alkanolamine AKN1 and the solvent are 100 wt% renewable and non-fossil source. in accordance with ASTM D6866-21, Method B.

The invention and the advantages thereof will be better illustrated by, but not limited to, the following examples given to illustrate the invention.

### EXAMPLES

### List of abbreviations:

DMAEA: 2-dimethylaminoethyl acrylate (CH₂=CH-C(=O)-O-(CH₂)₂-N(CH₃)₂)
MA: Methylacrylate (CH₂=CH-C(=O)-O-CH₃)
DMOH: Dimethylaminoethanol ((CH₃)₂N-(CH₂)₂-OH)
Hx: Hexane (CH₃-(CH₂)₄-CH₃)
DBTO: Dibutyl tin oxide ([CH₃-(CH₂)₃]₂Sn(=O))
Ptz: phenothiazine (N° CAS: 92-84-2)
GC-FID: Gas chromatography flame ionization detector
MeOH: Methanol
ICP-OES: Inductively coupled plasma - optical emission spectroscopy

### Example 1: Continuous synthesis of DMAEA

This section outlines the general procedure used for the examples; the various flow rates applied in the examples are summarized in Tables 1 and 2.

Two 1 L glass reactors (distillation devices DD1 and DD2), each equipped with a double jacket and a 50 cm high column with a 20 mm inner diameter, packed with 3 mm Dixon rings, are connected in series. A cooling condenser with glycol water at -10°C is placed at the top of the column of each distillation device DD1 and DD2. Agitation, in each distillation device DD1 and DD2, is provided by a mechanical stirrer with three blades inclined at 45°. The distillation devices DD1 and DD2 are initially filled with 240 g of a mixture of MA, Hx, DMOH, DBTO, and Ptz.

The reactors are preheated to 80°C, the top condensers are activated, and the stirrers are set to 500 RPM.

When the reaction mixture reaches 80°C, the continuous addition of reactants begins. Once the temperature of the top of the column drops to 50°C, the reflux rate is reduced, and distillate extraction starts.

A reflux control valve is used to collect the distillate, regulate the reflux rate, and indirectly control the column head temperature. The distillate is continuously collected from the upper section of the column, maintaining a temperature range of 48 to 53°C, corresponding to an azeotropic mixture of Hx/MeOH.

The reactor double jackets are progressively heated to 110°C. The reactor levels are increased to 800 mL and maintained throughout the synthesis, and the transfer between reactors (distillation devices DD1 and DD2) begins. After four complete renewals of the reactor volumes, the compositions are considered to have reached steady state, and samples are collected from each reactor (distillation devices DD1 and DD2). The reaction mixtures are analyzed using a GC-FID method. The reaction mixture from the final reactor (DD2) outlet is then distilled under heat and vacuum (distillation D3), using the same setup described earlier. Various fractions are collected until the final DMAEA fraction is obtained when the top column temperature reaches 78°C under pressures ranging from 40 mbar down to 5 mbar absolute. The DMAEA fraction is weighed and analyzed via the GC-FID method. The remaining residue is collected and weighed.

| | | Counter-example 1 | | Counter-example 2 | |
|---|---|---|---|---|---|
| Compounds | m reactors_initial (g) | Flowrate reactor DD1 (g/h) | Flowrate reactor DD2 (g/h) | Flowrate reactor DD1 (g/h) | Flowrate reactor DD2 (g/h) |
| MA | 142.4 | 118.7 | 0 | 118.7 | 0 |
| DMOH | 73.6 | 61.4 | 0 | 12.3 | 49.1 |
| HX | 20.8 | 17.3 | 0 | 17.3 | 0 |
| DBTO | 2.9 | 2.5 | 0 | 2.5 | 0 |
| Ptz | 0.2 | 0.1 | 0 | 0.1 | 0 |

| | | | | | |
|---|---|---|---|---|---|
| Table 1 - Quantities and flowrates of raw materials (counter-examples 1 and 2) | | | | | |

**Table 2 - Quantities and flowrates of raw materials (examples 1 and 2)**

| | | Example 1 | | Example 2 | |
|---|---|---|---|---|---|
| Compounds | m reactors_initial (g) | Flowrate reactor DD1 (g/h) | Flowrate reactor DD2 (g/h) | Flowrate reactor DD1 (g/h) | Flowrate reactor DD2 (g/h) |
| MA | 142.4 | 118.7 | 0.0 | 118.7 | 0.0 |
| DMOH | 73.6 | 46.0 | 15.3 | 30.7 | 30.7 |
| HX | 20.8 | 17.3 | 0.0 | 17.3 | 0.0 |
| DBTO | 2.9 | 2.5 | 0.0 | 2.5 | 0.0 |
| Ptz | 0.2 | 0.1 | 0.0 | 0.1 | 0.0 |

A gas chromatography GC Agilent 7820A is used with a DB-WAX UI column (30m length, 0.50µm inner diameter), starting at 80°C for 5 minutes, followed by a ramp-up of 4°C per minute to 122°C, held for 2 minutes, and then ramped up again at 35°C per minute to 240°C, which is held for 9 minutes. The injector is set to 250°C, with helium used as the carrier gas. A 1 µL sample is injected with a split ratio of 1:100. A flame ionization detector is used for detection.

Five standard samples with varying compositions of DMAEA, DMOH, MA, MeOH, and Hx are injected to establish calibration curves. The response factor obtained for DMAEA is applied to quantify heavier components observed after a retention time of 16 minutes.

The DMAEA conversion rate is calculated as follows: $DMAEA convertion rate \left(%\right) = \left(\frac{% DMAEA}{\left(%DMAEA+%DMOH+%Heavies\right)}\right) \times 100$

The complete results are summarized in Table 3 and 4. Adding a specific quantity of the total DMOH in the first reactor has a beneficial effect in reducing the heavies in both reactors and in the final reaction mixture before vacuum distillation D3.

However, a significant reduction in the DMOH quantity in the first reactor, while beneficial for reducing heavies, reduce the DMAEA conversion rate meaning the reaction takes more time. It is then possible to modify the ratio of DMOH added in the reactors depending on the desired goal.

The process according to the invention allows a reduction of the ratio between heavies' formation and DMAEA formation.

**Table 3 - Compositions of reactors and conversion rate of DMAEA at steady state (counter-examples 1 and 2)**

| | Counter-example 1 | | Counter-example 2 | |
|---|---|---|---|---|
| | Flowrate reactor DD1 (g/h) | Flowrate reactor DD2 (g/h) | Flowrate reactor DD1 (g/h) | Flowrate reactor DD2 (g/h) |
| % DMAEA | 25.0 % | 66.5 % | 6.3 % | 39.9 % |
| % DMOH | 15.20 % | 0.51% | 0.10 % | 12.16 % |
| % Heavies | 0.50 % | 1.10 % | 0.02 % | 0.20 % |
| % Conversion | 61.43% | 97.64% | 98.12 % | 76.35 % |
| 1000*(% Heavies/% DMAEA) | 20.0 | 16.5 | 3.2 | 10.0 |

**Table 4 - Compositions of reactors and conversion rate of DMAEA at steady state (examples 1 and 2)**

| | Example 1 | | Example 2 | |
|---|---|---|---|---|
| | Flowrate reactor DD1 (g/h) | Flowrate reactor DD2 (g/h) | Flowrate reactor DD1 (g/h) | Flowrate reactor DD2 (g/h) |
| % DMAEA | 18.8 % | 67.2 % | 12.5 % | 66.20 % |
| % DMOH | 2.20 % | 0.45 % | 0.20 % | 2.00 % |
| % Heavies | 0.10 % | 0.30 % | 0.10 % | 0.20 % |
| % Conversion | 89.07% | 98.80% | 97.66% | 89.00% |
| 1000*(% Heavies/% DMAEA) | 5.3 | 4.5 | 8.0 | 3.0 |

## Claims

1. Process for the continuous synthesis of an amino alkyl (meth)acrylate AAE2 wherein the process comprises continuous transesterification reactions carried out in a first distillation device DD1 and in a second distillation device DD2, wherein the first and the second distillation devices DD1 and DD2 are arranged in series,
wherein the process comprises the following steps:
1/ adding to the first and the second distillation devices DD1 and DD2, a mixture M1 comprising an alkyl(meth)acrylate ester AAE1, a solvent S1, an alkanolamine AKN1 and a catalyst C1;
2/ transesterification reactions, in the first and second distillation devices DD1 and DD2, of the alkyl(meth)acrylate ester AAE1 with the alkanolamine AKN1 while continuously distilling an azeotropic mixture of solvent S1 and an alkyl alcohol formed during the transesterification reactions,
wherein the transesterification reactions afford an amino alkyl(meth)acrylate AAE2 and are carried out at a temperature of between 40 and 120°C; and,
wherein the first distillation device DD1 is continuously fed with a mixture M2 comprising the alkyl(meth)acrylate ester AAE1, the solvent S1, the alkanolamine AKN1 and the catalyst C1; and,
wherein the second distillation device DD2 is continuously fed with the alkanolamine AKN1; and,
wherein the weight ratio between the alkanolamine AKN1 fed to the first distillation device DD1 and the alkanolamine AKN1 fed to the second distillation device DD2 is comprised between 0.4:1 and 6:1, based on the total amount of alkanolamine AKN1 fed in the transesterification reactions; and,
wherein during the transesterification reaction, an overflow of the first distillation device DD1 fills the second distillation device DD2.

2. Process according to any one of the preceding claims, ***wherein*** the transesterification reaction in the first distillation device DD1 lasts between 30 minutes and 720 minutes.

3. Process according to any one of the preceding claims, ***wherein*** the transesterification reaction in the second distillation device DD2 lasts between 30 minutes and 720 minutes.

4. Process according to any one of the preceding claims, ***wherein*** the alkyl(meth)acrylate ester AAE1 is methyl(meth)acrylate.

5. Process according to any one of the preceding claims, ***wherein*** the at least one alkanolamine AKN1 is a tertiary amine HO-NR₁R₂ wherein R₁ and R₂ are alkyl groups comprising, independently from each other, from 1 to 8 carbon atoms.

6. Process according to any one of the preceding claims, ***wherein*** the at least one alkanolamine AKN1 is dimethylaminoethanol.

7. Process according to any one of the preceding claims, ***wherein*** the catalyst C1 is dibutyltin oxide.

8. Process according to any one of the preceding claims, ***wherein*** the transesterification reaction is carried out in the presence of a quantity of catalyst C1 of between 0.1 and 150 mmol per mol of the at least one alkanolamine AKN1.

9. Process according to any one of the preceding claims, ***wherein*** the at least one alkyl (meth)acrylate ester AAE1 and the at least one alkanolamine AKN1 have a molar ratio of between 1:5 and 5:1.

10. Process according to any one of the preceding claims, ***wherein*** the at least one solvent and the total amount of (i) the at least one alkanolamine AKN1, (ii) the at least one alkyl(meth)acrylate ester AAE1 and (iii) the catalyst C1 have a weight ratio of between 0.0001:1 and 0.3:1

11. Process according to any one of the preceding claims, ***wherein*** at least one of the at least one alkyl (meth)acrylate ester AAE1, the at least one alkanolamine AKN1 and the at least one solvent originates from a feedstock comprising recycled material or materials of biological origin, the biobased carbon content is measured in accordance with ASTM D6866-21, Method B.
